# EUROPEAN PATENT SPECIFICATION

(11) **EP 3 271 454 B1**
(45) Date of publication and mention of the grant of the patent: **04.10.2023**
(21) Application number: 16726479.5
(22) Date of filing: 21.03.2016
(51) Int. Cl.: C12N 5/074, C12M 1/26, C12M 1/00, C12N 5/077, A61K 35/28

(54) **SYSTEM AND METHOD FOR PROVIDING ISOLATED CONCENTRATED MULTIPOTENT STROMAL CELLS FOR ADMINISTRATION**
SYSTEM UND VERFAHREN ZUR BEREITSTELLUNG ISOLIERTER KONZENTRIERTER MULTIPOTENTER STROMAZELLEN ZUR VERABREICHUNG
SYSTÈME ET PROCÉDÉ PERMETTANT DE FOURNIR DES CELLULES STROMALES PLURIPOTENTES CONCENTRÉE ISOLÉES POUR L'ADMINISTRATION

(30) Priority: 20.03.2015 EP 15160195
(43) Date of publication of application: 24.01.2018
(73) Proprietor: Educell D.O.O., 1236 Trzin (SI)
(72) Inventor: VEBER, Matija, 1236 Trzin (SI); BARLIC, Ariana, 1236 Trzin (SI); KNEZEVIC, Miomir, 1236 Trzin (SI); STRBAD, Marko, 1236 Trzin (SI)
(74) Representative: Hoefer & Partner Patentanwälte mbB
(86) International application number: PCT/EP2016/000493
(87) International publication number: WO 2016/150566

(56) References cited:
- EP-A1- 1 947 170
- WO-A1-2014/093845
- US-A1- 2013 129 688
- US-A1- 2014 227 240
- KINYA ITO ET AL: "A Novel Method to Isolate Mesenchymal Stem Cells from Bone Marrow in a Closed System Using a Device Made by Nonwoven Fabric", TISSUE ENGINEERING PART C: METHODS, vol. 16, no. 1, 1 February 2010 (2010-02-01), pages 81-91, XP055080730, ISSN: 1937-3384, DOI: 10.1089/ten.tec.2008.0693
- S Ciuffi ET AL: "P3 -HUMAN MESENCHYMAL STEM CELLS: ISOLATION AND CONCENTRATION FROM BONE MARROW USING THE REGENKIT DEVICE", Clinical Cases in Mineral and Bone Metabolism, 1 September 2010 (2010-09-01), pages 207-240, XP055203789, Retrieved from the Internet: URL:http://www.ncbi.nlm.nih.gov/pmc/articl es/PMC3213771/pdf/ccmbm7_3p209.pdf [retrieved on 2015-07-21]
- 1 Jones ET AL: "Rapid Intraoperative Concentration of Mesenchymal Stem Cells from Bone Marrow Aspirate", , 24 February 2009 (2009-02-24), XP055203794, Retrieved from the Internet: URL:http://www.ors.org/Transactions/55/091 8.pdf [retrieved on 2015-07-21]

## Description

### Field of the invention

The present invention is concerned with a system as defined in the claims for isolating, optionally expanding, and concentrating multipotent mesenchymal stromal cells (MSCs) and with a method as defined in the claims of obtaining MSCs ready for administration to a patient in need of MSCs to repair tissue damage or to treat a disease or condition with MSCs.

### Background

Multipotent stromal cells also designated as mesenchymal stromal cells or mesenchymal stem cells and abbreviated as MSCs are self-renewing, multipotent progenitor cells with multilineage potential to differentiate into cell types of mesodermal origin, such as adipocytes, osteocytes, and chondrocytes, tenocytes, and myocytes. In addition, MSCs can migrate to sites of inflammation and exert potent immunosuppressive and anti-inflammatory effects through interactions between lymphocytes associated with both the innate and adaptive immune system. Furthermore, genetic modification of MSCs to overexpress antitumor genes has provided prospects for clinical use as anticancer therapy.

Due to the MSCs having the ability to differentiate across various lineages beyond the conventional mesodermal lineages, they can be applied in regenerative medicine and tissue repair. Therefore, MSCs have been used in medical fields such as orthopedics as site-specific delivery vehicles to repair cartilage, bone, tendon, marrow stroma, muscle, and other connective tissues since the 1990s.

MSCs possess further unique characteristics that make them attractive therapeutic agents for treatment of various diseases. It has been shown that MSCs can provide therapeutic benefit through the secretion of soluble factors to induce an immunomodulatory environment. Furthermore, MSCs have the capacity to migrate toward sites of injury and tumor microenvironments. Therefore, MSCs can serve as delivery vehicles for targeted therapy. In a clinical setting, MSCs are being explored in trials for various conditions, including orthopedic injuries, graft versus host disease (GvHD) following bone marrow transplantation (BMT), cardiovascular diseases, autoimmune diseases, and liver diseases. Furthermore, genetic modification of MSCs to overexpress antitumor genes has provided prospects for use as anticancer therapy in clinical settings [1].

While MSCs are most commonly isolated from bone marrow (BM), they can also be isolated from other tissues including adipose tissue, placenta, amniotic fluid, and umbilical cord blood. The number of MSCs in bone marrow, however, is low. MSCs represent 0.001-0.01 % of BM mononuclear cells or lower. The optimal dosage of MSCs in therapeutic applications often is higher and is dependent upon the type of therapy, however, an amount from 0.5 to 5 * 10⁶ MSCs per kg body weight is generally useful. Direct collection of such a large number of MSCs from bone marrow is often not possible. Therefore, it is often necessary to expand isolated MSCs ex vivo to obtain a sufficient number for therapeutic approaches [2]. Although it is not difficult to isolate and expand MSCs ex vivo, there are conditions to be fulfilled if those MSCs are reintroduced into a patient.

As a general preparation protocol for clinical use, the bone marrow cells aspirated from bone marrow undergo first an ex vivo separation (MSC isolation) and then an MSC expansion.

The conventional isolation method is carried out by density centrifugation with lymphocyte separation medium (LSM). As a general outline, the bone marrow is diluted with saline solution, layered over LSM and the mononuclear cell layer is then collected after centrifugation. The isolated MSCs are then harvested, expanded, and differentiated if requested by the clinical application, in cell culture plates with suitable medium and supplement by means of standard, well established methodologies.

Major weaknesses of this conventional method are given by the complexity of the method and the high risk of sample contamination due to manipulation. In general, culture medium must be changed continuously in the culture, non-adherent cells must be removed from it, and the whole process is time-demanding.

Advancements have been made to withdraw and/or isolate and/or expand cells in controlled and isolated environments, as can be seen in the extensive literature in this field.

Among the various publications, Ciuffi S. et al. (Clinical Cases in Mineral and Bone Metabolism 2010, 7(3), 207-240) describes a method of selection and enrichment of autologous mesenchymal and hematopoietic stem cell fractions from bone marrow by means of the RegenKit^{®}. The method provided in Ciuffi S. et al. does not comprise a concentration unit as defined in the present invention.

Jones S.C. et al. (Poster No. 918 • 55th Annual Meeting of the Orthopaedic Research Society) reports on the intraoperative use of CellMAX^{™} Bone Marrow Aspirate Concentrate System to produce a concentrated cell-rich fraction from bone marrow harvested in a clinical setting. The system provided in Jones S.C. et al. does not comprise a concentration unit as defined in the present invention.

Both procedures, however, need a separated operation of centrifugation, where the product must be taken out from the controlled environment and be poured into a centrifuge, thus having a risk of contamination.

As the MSCs shall be provided for administration to a patient, the risk for contamination has to be kept as low as possible and the isolation and expansion method shall be suitable and safe to not introduce contaminants into the patient. For this reason, care has to be taken that all steps involving the manipulation of the MSCs are carried out in environmentally controlled areas. This requires the consequent need of sophisticated systems, tightly controlled and maintained and highly specialized personal.

Furthermore, extended tissue culture should be avoided, as this could cause potential hazards, including contamination and malignant transformation with chromosomal alterations. Prolonged culture could also cause changes of gene expression, which could be detrimental for the therapeutic potential of the cells. Therefore, a fast and high yield growth of MSCs is needed to allow for generation of a sufficiently large population of MSCs for clinical use and for avoiding the potential collateral hazards.

US 2014/0141505 and related patents (i.e. EP 24 50 431, JP 2011/010582, US 2009/0142835) disclose stem cell separating material and a method of separation of stem cells, wherein a specific material made from polymeric fibres is used to separate stem cells from other cells present in blood. A device comprising this material is used to separate cells by contacting a fluid with the material whereby MSCs are captured by the material and red blood cells flow through. The cells enriched with this system can be transferred to a culture system to expand the cells.

Also in the same family of patents, EP 1947170 has the object to provide fiber material and a filter for separating stem cells, which are capable of selectively separating and recovering stem cells from body fluids or biological tissue-derived treated fluids. The material for separating stem cells can be packed in a container having a fluid inlet port and a fluid outlet port, thus separating and producing a multipotent cell fraction. The system provided in EP 1947170 does not comprise a concentration unit as defined in the present invention.

The above approaches have been exhaustively reported and summarized by Ito K. et al. (Tissue Engineering Part C 2010, 16(1), 81-91). A device containing polymeric nonwoven fabric filter selectively traps bone marrow stem cells based on affinity, not cell size. The cells are then recovered by the retrograde flow and can be directly used for clinical application. The systems discussed in Ito K. et al. do not comprise a concentration unit as defined in the present invention.

By means of these systems, the aspirated bone marrow is injected into a Bone Marrow MSC Separation Device, which had been previously filled with saline solution. After washing with fresh saline to remove red blood cells and platelets, the trapped cells are removed through the device into a closed collection bag, where they will then be harvested, by back flushing culture medium (i.e. Dulbecco's modified Eagle medium with fetal bovine serum as a supplement). The same principle can be adapted to other biological fluids.

This methodology is completed in a closed system, as highlighted in the above mentioned article of Ito K. at al. "For clinical use, it is important to develop a way to isolate BMSCs from bone marrow in a closed system without centrifugation*".* The risk of external contamination given by open systems such as density centrifugation and standard cell culture is therefore reduced. However, a problem with such devices is that although all of the MSCs obtained would be desirable to be administered, the whole volume of liquid containing MSCs cannot be administered to the patient. The Bone Marrow MSC Separation Device provides MSCs suspended in about 50 ml of liquid, if not more, at the end of harvesting. A volume of about 50 ml or more is however impractical or even impossible to be administered.

In some therapeutic approaches, it is necessary to have a small volume with an available number of MSCs available for direct administration, optionally directly during a surgical operation. In this case, it is the object of the present invention to provide a method, wherein MSCs can be isolated in a fast and safe manner.

For example, in the medical field of orthopedics, delivery of cells into a joint to treat skeletal injuries is taken daily in clinical practice in general by percutaneous injections and arthroscopic placement. The orthopedic surgeon should not inject more than 10 ml, possibly 5-6 ml, to comply with the clinical protocol for treatment of various skeletal conditions, in particular for osteoarthritis, osteochondral lesions and cartilage repair.

Furthermore, if the isolated and expanded MSCs are not for immediate administration, they need to be cryo-conserved. To ensure an optimal viability and functionality upon thawing of the samples, the concentration of MSCs during cryopreservation is proposed to be optimal at about 0.5 to 1 × 10⁶ MSCs/ml [4].

If cryo-conservation and conventional cell expansions of MSCs in sterile conditions is necessary for such preparations, this has to be done by carrying out all steps in environmental controlled area (i.e. class A), with consequent need of sophisticated systems, tightly controlled and maintained, and highly specialized personnel.

Beyond the concentration of bone marrow aspirate or harvested cells by a separate step of centrifugation with one of the well-known available methods, US 2014/0227240 reports in Example 2 optionally a concentration by a hemoconcentrator. No further information is given. Therefore, US 2014/0227240 does not disclose a system or method comprising a concentration unit as defined in the present invention.

US 2013/0129688 A1 is concerned, *inter alia,* with a method of concentrating cells and teaches alleged advantages of tangential flow filtration compared to dead-end filtration.

WO 2014/093845 describes a system for isolation and concentration of stem cells from cell-containing biological sample. The system provided in WO 2014/093845 does not comprise a concentration unit as defined in the present invention. The cell separation is carried out by means of a syringe that contains a separation medium. The concentration uses a principle of tangential flow filtration by pushing the cell suspension through hollow filter with syringes.

Results of cell isolation from blood and blood components are only provided in WO 2014/093845. This document does not present any quantitative data about isolation of mesenchymal/stromal cells (MSCs). Microscope images of an MSC culture isolated with the system of WO 2014/093845 are presented without quantification of the efficacy of the isolation system.

The present inventors have repeated the experiments of cell isolation as described in WO 2014/093845 but got unsatisfactory results for the isolation of MSCs (<65%) versus other more efficient systems (e.g. Kaneka, with >80% recovery using analogous media). Moreover, the separation medium in the syringe also caused faster sedimentation of unwanted cells (impurities).

The inventors have also repeated the concentration of blood components and MSCs suspensions as in WO 2014/093845 by hollow fibre filters usually present in hemo-filtration and plasma separation membranes. When blood showed a fast concentration process to a low volume, satisfactory recovery and negligible side effects, the outcomes with MSCs were unsatisfactory, probably due to the different characteristics of the two biological samples. It was found that with this process a lower recovery is obtained and by partial clogging of filters the process becomes slower.

In a clinical setup, any requirement of additional equipment adds additional costs and occupies working area in usually very crammed operating theatres. Thus, it is another object to provide a simple yet controlled concentration process, which can be used when preparing cell suspensions for therapeutic purposes.

Hemoconcentrators and plasma separators usually require dedicated pumps to control the flow rates of fluids through the filters. Thus, another object is to avoid the use of such devices.

Pumps could be replaced with syringes and the cell suspension could be manually pushed through a hollow fibre filter, but this is a highly uncontrolled process, and is subject to the skills of an individual operator. An improper handling can easily result in excessive flow rate and trans-membrane pressure, which can damage both the cells and the hollow fibre membrane. Thus, a further object was to provide a process that avoids damage for cells.

Summarizing, it was the object of the present invention to provide MSC preparations and concentrates having a high number of cells in a small volume, i.e. that have a high cell density, that can be obtained in high quality and quantity (recovery) without the risk of external contamination, and that can be used directly for administration to a patient. Furthermore, the need for working in an environmental controlled area should be avoided, and a fast process would be preferred. Moreover, the use of devices as necessary in the prior art methods should be overcome and a process using mild conditions shall be provided.

All these objects are solved by the method and the device of the present invention as defined in the claims.

Surprisingly, the inventors found that hollow fibre filters, normally used as tangential flow filters, can be efficiently used also as dead-end filters for the purpose of the cell concentration. This simplifies a flow regime of fluids through the filter and removes the flow of the retentate out of the set-up. Moreover, in this set-up a critical parameter, a trans-membrane pressure, can be easily controlled only by the pressure difference between feed suspension and the filtrate.

One way to control trans-membrane pressure is by defining an exact height difference between the reservoir of inlet solution and the reservoir of the filtrate. The trans-membrane pressure can be directly defined by the difference in hydrostatic pressure of the fluid between the two reservoirs. The height difference can be easily limited by the length of the tubing between the reservoirs and the filter. The whole system can be supported, such as hung on a common infusion stand and all crucial filtration parameters can be defined without any intervention of personnel or need for additional equipment.

When using a hollow fiber filter in a dead-end filtration set-up, the relatively large surface area of the hollow fibre filters in relation to their volume can be exploited. Large surface area prevents the cells from clogging up the pores in the filters and the small volume enables the cells could be concentrated to a small enough volume.

The flow through design of such filters can also facilitate in a more efficient retrieval of the cells from the filter. By using dead-end filters, cells could be retrieved only through retrograde flow through the filter. Additionally, use of hollow fibre filters also allows for washing of cells in the direction perpendicular to the surface of the membrane. With the combination of retrograde flow of fluid and air flow within the hollow filters a technique has been established which results in high yield of the cell concentration process.

The combination of efficient separation column and cell concentrator as defined in the claims results in high cell yields, which are required for the clinical applications and at the same time enables the operation in a closed system without the need of any additional equipment in a timely manner.

The present invention provides a completely closed system and a method as defined in the claims which allow isolation, separation, concentration, optionally expansion, and, if necessary, preservation of MSC in one system which overcomes the problems with external contamination and provides a preparation with high separation yield and high cell density. The preparation obtained can be directly applied to a patient as it has a small volume and is safe. The device allows carrying out the whole procedure under sterile conditions without using an environmental controlled area so that the method can be carried out in an operating room or an emergency room or in a doctor's surgery or practice.

The system and the method of the present invention as defined in the claims provide an MSC concentrate that has a concentration suitable for direct administration or for cryopreservation or cryo-conservation a high yield from a biological sample. The biological characteristics and properties of the obtained MSCs are comparable to MSCs obtained with conventional methods, whereas the density and sterility of the obtained preparation is superior to preparations obtained with conventional methods. As the system of the present invention is a closed system, it is ensured that a contamination of the MSCs does not occur.

Furthermore, the system and the method as defined in the claims are effective and easy to handle. The parts necessary for processing the sample can be either assembled and sterilized or can be provided as complete system in sterile form. The sample is introduced at the port, processed within the system and harvested at the outlet of the concentration unit. No external manipulations are made during processing within the system. The system allows to work under sterile conditions. After harvesting the cells, the system is either discharged or can be sterilized for a next procedure. The harvested cells can be used directly on the spot for the same patient, can be preserved for example by cryopreservation and then administered, or can be administered to a different person directly after harvest, after expansion or after storage.

The present invention provides, in a closed system, a combination of a separation unit and a concentration unit as defined in the claims. The system allows isolating MSCs from a biological sample, such as bone marrow or from other sources, optionally expansion of MSCs to obtain a high number of MSCs, and concentration of MSCs to have a small volume with a high density of cells in a sterile suspension, which can be directly administered to the patient or can be cryopreserved. The characteristics and properties such as viability, multipotency and functionality of MSCs obtained with the present system are not adversely affected and are at least as good as for MSCs obtained by a conventional system or method. Therefore, the characteristics and properties of the MSCs should be at least similar to those obtained with known methodologies for therapeutic purposes.

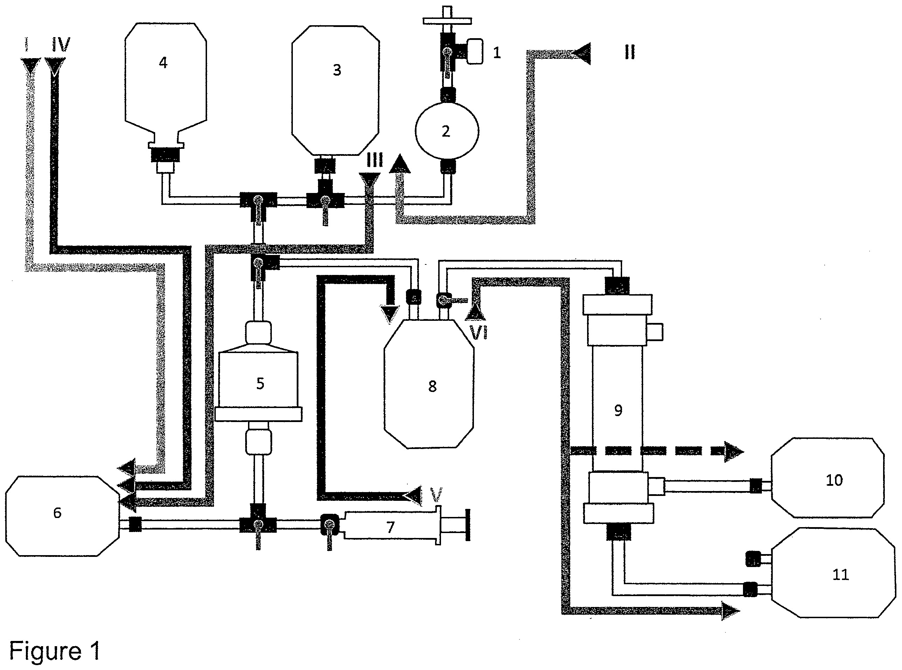

An example of a system of the present invention combining an MSC separation unit and a concentration unit is shown in Figure 1. Both are combined in a manner such that the system as a whole is closed and, therefore, not prone to contamination. With this system and this method it is possible to produce an MSC sample with clinical characteristics not met before. The MSCs obtained with the system and method of the present invention had a concentration suitable for direct administration or for cryo-conservation. The characteristics and properties of the obtained MSCs were comparable to MSCs obtained with conventional methods.

The sample comprising MSCs is a biological fluid and can for example be obtained from bone marrow, amniotic fluid, or umbilical cord blood.

The first part of the system of the present invention is a separation unit as defined in the claims, which comprises an inlet port for introduction of a sample, a column comprising separation means for separating MSCs comprised in a sample, and an MSC outlet.

The inlet port and the outlet of the concentration unit are the only parts of the whole system that are in contact with the environment, whereas the rest of the internal system has no direct contact to the environment and is not manipulated externally during processing.

The inlet port for introduction of a sample is a port as usually used for separation columns, for example an injection port. The introduction port is configured to ensure tightness and thereby to avoid external contamination when introducing the sample.

Optionally, downstream from the port, preferably close to the inlet, the system can comprise a particle filter to separate solid particles, like bone fragments, that might have been withdrawn together with the cells when obtaining a sample comprising MSCs.

The inlet port and the optional filter can be fluidly connected such as by sterile lines, such as tubing, with a sample vessel (3) for receiving the sample comprising the MSCs.

The separation unit comprises a separation column with a separation means. The separation means can be in the form of a separation filter, separation beads, or a separation gel for separating cells. These means work on the principle of chemical and/or physical, or physico-chemical affinity between a separation means (e.g. polymeric filter) and a biological sample. It has been found and experimentally shown by the inventors that separation of MSCs from a biological sample provides a higher yield and superior properties of the cells when compared to other methods of separation as used in the prior art. Separation filters for separating MSCs are well-known and are commercially available. The separation means or separation filter can for example be made from a material as described in EP 24 50 431, i.e. a filter material that captures MSCs whereas red blood cells flow through.

The captured MSCs can then be harvested for example by applying a washing solution and/or by back-flushing, with a liquid such as an isotonic aqueous salt solution or culture medium. It is also possible to use a separation column filled with beads or a gel for separating MSCs.

For enriching MSCs, for example aspirated bone marrow can be injected via an inlet port into the Bone Marrow MSC Separation Device, which has previously been filled with saline solution. After washing with fresh saline to remove red blood cells and platelets, the trapped MSCs are harvested by back flushing culture medium, which is for example Dulbecco's modified Eagle medium optionally with supplement as known in the art.

It has been found, that using a separation unit as mentioned above provides an improvement over the conventional isolation procedure using LSM, as a significantly faster growth of MSCs is obtained, whereas the quality of the MSCs, such as character and multi-potency, is equivalent to those obtained by the conventional method [3].

The MSC rich fraction obtained from the separation unit is then transferred via communication means to a concentration unit to reduce the volume of this fraction.

Communication means are provided between the separation unit and the concentration unit to allow the transfer of the MSC rich fraction without contamination. The term "communication means" shall comprise means like tubes or lines, storage devices like bags or containers, and feeding or transport means, like pumps. All these means are those that usually are used in such systems. All means and units are made from material that is inert i.e. does not react with any of the components used therein and optionally is sterilisable. Moreover, feeding or transport means are those that are usually used in this field, for example pumps that can be set and operate automatically without the need for external adjustment. Containers or bags for receipt and storage of fluids, such as washing fluids and elution fluids, for waste storage etc. are included in the closed system and provide all necessary liquids in predetermined amounts so that no external operation is necessary during the processing.

The concentration unit is a unit for reducing the volume of the enriched MSC fraction. It can be a hollow fiber dead-end ultra-filtration unit that allows reducing the volume from 1 to 0,5 v/v to 1 to 0,05 v/v.

Concentration devices are known. It has been found by the inventors of the present invention that ultra-filtration devices that are routinely used for concentration of blood in cardiopulmonary bypasses (CBP) are particularly useful. Such a unit is biocompatible and can be used in the present system. The concentration unit comprises an ultra-filtration system and an MSC receiving vessel. The vessel comprises the enriched fraction with reduced volume and can either be used directly or can be cryo-preserved.

Ultra-filtration has been used since the 1970s and has been found to be an excellent method for treating severely hemodiluted patients by concentrating blood remaining in the extracorporeal circuit after CPB and by removing extra-corporally excess body fluid from the intravascular space of patients in renal failure undergoing open-heart surgery safely. Moreover, ultra-filtration has been used to concentrate blood in normal patients diluted by over-hydration and to serve as an effective method for blood conservation through the preservation of platelets and coagulation factors. More recently, extracorporeal use of blood ultra-filtration has been employed to reduce post-CPB inflammatory responses and immunologic activation, and to provide the additional benefit of moderating temperature elevations in the postoperative period due to removal of circulating pyrogen.

The primary principle governing ultra-filtration is the selective separation of plasma water and low-molecular weight solutes from the intravascular cellular components and plasma proteins of blood, using a semi-permeable membrane filter. The driving force for ultra-filtration is provided by the hydrostatic pressure differential occurring across the ultra-filtration membrane. The application of a negative pressure on the effluent side of the membrane or the use of an increased perfusion pressure applied to the blood site of the membrane results in improved solute and fluid filtration.

It has now been found that ultra-filtration is a particularly useful method for reducing the volume of a cell fraction.

Standard ultrafiltration procedures require dedicated equipment in order to ensure filtration parameters or conditions, respectively, which would not or hardly present any risk for the cells. There is a need for a simple controlled concentration process without the use of additional equipment.

Ultra-filtration units that are available for this purpose are commercially available, for example Plasmaflo^{™} (Asahi Kasei), Fresenius HF models (Fresenius), Maquet BC models (Maquet), Minntech HPH models (Minntech), Sorin DHFO and SH models (Sorin), and Terumo HC models (Terumo).

Thus, it was surprisingly found that MSCs can be isolated and harvested in a fully closed sterile system, which yields MSCs fast and in excellent quality and high number and the resulting final volume of MSCs suspension is not higher than 10 ml. In one embodiment, a combination of Bone Marrow MSC Separation Device or a similar system with a hemoconcentrator is used and an example is schematically shown in Figure 1. The new system overcomes the disadvantages of the systems and methods available in the prior art.

By means of this novel and inventive closed system combining an isolation subsystem with a concentration subsystem, an MSC suspension with a volume not higher than 10 ml can be obtained. The suspension is sterile and the characteristics of the MSCs are the same after isolation and harvesting in the Bone Marrow MSC Separation Device.

Moreover, if the number of MSCs obtained is sufficient, expansion is not needed in the case of a single-step clinical intervention. In this case, the system allows obtaining a small volume of suspension ready for administration in a fast manner, which is then directly injected into the patient, without the need for external processing.

In a further embodiment, the system of the present invention additionally comprises an expansion unit to allow amplifying the MSCs obtained from a patient. In many therapeutic applications, the number of MSCs that is obtained when a sample is withdrawn from bone marrow, is insufficient for therapeutic effect. For these cases, a system is provided, that in addition to a separation unit and a concentration unit comprises an expansion unit within the closed system. This system also allows obtaining MSCs in high number in a low volume without the risk for contamination.

The expansion unit comprises a vessel for growing cells, which is in fluid communication on one side with the separation unit and on the other side with the filtration unit. The growth vessel contains or is fed with an expansion medium. Such medium is well-known to the skilled person. Media that are useful for growing MSCs are available on the market and can be used in this system. One example is Dulbecco's modified eagle medium with serum supplementation.

Feeding means such as a pump can be provided to allow transport of the enriched fraction to the harvesting bag and from the harvesting bag to the concentration unit.

The system of the invention is therefore highly flexible and can be used
a. to isolate MSCs and to directly concentrate the isolated MSCs to a suitable small volume of cell suspension without an expansion step in the context of a single clinical surgical procedure or
b. to isolate MSCs, to expand the isolated MSCs after harvesting, and to concentrate the MSCs to a suitable small volume of cell suspension in a closed environment maintaining sterile conditions;

The system of the present invention can be used by introducing a sample comprising MSCs and a fraction enriched with MSCs and, compared to compositions comprising MSCs as known in the prior art, reduced volume is obtained.

With the system of the present invention, it is possible to obtain a sterile MSC suspension with a very low volume, such as 10 ml, which however, comprises a high number of MSCs that are useful for therapeutic administration. It was found, that when using the system of the present invention for separating, optionally expanding, and harvesting MSCs, the characteristics of the cells are the same after isolation and harvesting as before and as cells that have been grown in a system of the prior art.

The present system has the advantage that it is possible to provide a MSC suspension from a sample that has been obtained from a patient in short time and in easy manner. This allows using the system in the surgical room so that the surgeon can withdraw a sample of bone marrow from a patient, can separate, and concentrate the MSCs and can apply them in the same surgical operation to the patient because of the low volume.

There is also the option to amplify the MSCs obtained, if the number is too low, in the same system and to apply the concentrate thereafter. Thus, if the number of MSCs that have been withdrawn and have been separated is not enough, the cells can be expanded within the system.

The invention is also explained with reference to Figure 1, which shows an exemplary system according to the invention for separating/isolating and concentrating MSCs.

In Figure 1 a system is schematically shown which comprises an injection port (1) for receiving a sample comprising MSCs. The injection port is configured to ensure tightness and thereby to avoid external contamination when injecting the sample. Optionally, immediately downstream from the injection port, the system comprises a filter (2) to separate possible solid particles contained in the sample comprising MSCs.

The sample comprising MSCs is a biological fluid and can be obtained from bone marrow, amniotic fluid, or umbilical cord blood. If the sample is a bone marrow aspirate, the solid particles can originate from tissue aspirated during the bone marrow aspiration.

The injection port and the optional filter are fluidly connected by sterile lines, such as tubing, with a sample vessel (3) for receiving the sample comprising the MSCs.

The system further comprises a wash vessel (4) for storing wash solution. The wash solution is usually a saline solution, such as a 0.9 % sodium chloride solution or a phosphate buffered saline (PBS).

The wash vessel is fluidly connected by sterile lines with the separation and isolation device, such as a separation column (5) for isolating the MSCs comprised in the sample. The separation column comprises nonwoven biocompatible fabric filter on its internal surface.

The separation column (5) is fluidly connected to a waste vessel (6) for storing used wash solution, which has been transferred from the wash vessel via the separation column to the waste vessel.

The separation column (5) is also fluidly connected to the sample vessel (3). The lines fluidly connecting wash vessel (4) and separation column (5) and the lines fluidly connecting sample vessel (3) and separation column (5) can share a common region. In this case, the lines are joined by a three-way stopcock upstream of the separation column (5). Depending on the position of the stopcock, the fluid connection is open for fluid communication between sample vessel and separation column. In the other position, the fluid connection is open for fluid communication between wash vessel and separation column

The separation column (5) is also fluidly connected to a harvest vessel (7) for storing a harvesting solution. The harvesting solution is a culture medium suitable for culturing MSCs. An exemplary culture medium is Dulbecco's modified eagle medium (DMEM). DMEM can be supplemented as is common in this field, for example with L-Glutamine, antibiotics, plasma and/or serum.

The lines fluidly connecting waste vessel (6) and separation column (5) and the lines fluidly connecting harvest vessel (7) and separation column (5) can share a common region. In this case the lines are joined by a three way stopcock downstream (in relation to the fluid flow of the wash solution) of the separation column (5). Depending on the position of the stopcock, the fluid connection is open for fluid communication between separation column and waste vessel. In the other position, the fluid connection is open for fluid communication between harvest vessel and separation column.

If an expansion of the isolated MSCs is desired, indicated, or necessary, the system of the invention can optionally comprise an expansion vessel (8) for receiving the harvested isolated MSCs. If expansion is indicated, the expansion vessel comprises a culture medium suitable for MSC expansion. The culture medium can be substantially the same medium has the culture medium used as harvesting solution. Furthermore, growth factors can be comprised in the expansion medium. The expansion vessel is fluidly connected with the separation column.

The system further comprises an ultra-filtration column (9) for concentrating the harvested MSCs. The ultra-filtration column is fluidly connected either directly with the separation column, if no expansion vessel is present, or with the separation column via the expansion vessel.

The system further comprises an MSC vessel (11) for receiving the concentrated isolated MSCs. The MSC vessel is fluidly connected with the ultra-filtration column (9).

The system can further comprise a further waste vessel (10) for receiving excess liquid removed from the sample in the ultra-filtration column (9), and lines fluidly connecting the ultra-filtration column (9) and the waste vessel (10).

The fluid connections providing fluid communication between the components of the system ensure that the system is a fully closed system comprising only sterile components.

The system of the present invention as described above can be used in a method for obtaining isolated and concentrated MSC in the following way.

The wash vessel is filled with a washing or priming solution. The separation column is then primed for use by transferring the wash solution from the wash vessel to the separation column. The used wash solution is then stored in the waste vessel (6).

In the next step, a sample comprising the MSCs, such as bone marrow aspirate, is injected into the injection port (1). The optionally filtered sample is then transferred into the sample vessel (3).

The sample vessel is for temporary storage of the optionally filtered sample comprising MSCs in a buffering container and, consequently, to allow a successive controlled and complete transfer of sample into the separation column.

In the next step, the sample fluid is transferred from the sample vessel into the separation column. After entering the separation column, the MSCs comprised in the sample will attach to the internal surface of the column, and will thereby be trapped. The components of the initial sample, which are not trapped in the separation column, such as blood residuals, white blood cells, red blood cells, are directly transferred to the waste vessel (6) and are completely removed from the separation column by flowing wash solution via the separation column to the waste vessel.

In the next step, the trapped MSCs in the separation column are harvested by flowing harvesting solution contained in the harvest vessel (7) from the harvest vessel, such as a syringe, via the separation column to the expansion vessel. The harvesting solution detaches and "frees" the trapped MSCs in the separation column. If no expansion is necessary or indicated, the harvested cells can either be directly transferred from the separation column to the ultra-filtration column (9), or by passing through the expansion vessel (8) without undergoing an expansion step within the expansion vessel.

If an expansion step is indicated, the MSCs are then subjected to expansion condition within the expansion vessel until a desired number of MSCs suitable for a further clinical application is reached.

The sample fluid comprising the isolated and optionally expanded MSCs transferred to the ultra-filtration column are then transferred through the ultra-filtration column. The concentrated suspension of MSCs is collected in the MSC vessel (11) and usually has a small volume of about 5 to 10 ml. Excess liquid removed from the sample fluid during ultra-filtration can be collected in the waste vessel (10).

The small volume of MSCs in concentrated suspension is then ready for either cryo-conservation of for administration to the patient in the context of a clinical protocol and procedure.

The MSCs obtained can be used as MSCs obtained with methods disclosed in the prior art.

Another aspect of the present invention is the use of the MSCs obtained with the system of the present invention or the method of the present invention, respectively. The MSCs obtained according to the present invention can be used as MSCs obtained with methods of the prior art. The MSCs can be used for autologous therapy as well as allogenic therapy. Whether autologous or allogenic preparations are useful depends from the therapy.

MSCs are able to differentiate into mesodermal cell types/tissues, such as osteoblasts, osteocytes, chondrocytes, myoblasts, myocytes, stromal cells, tenocytes, adipocytes and other connective tissue cells. Thus, MSCs can be used as therapeutic agent to treat patients in need of such cells.

On the other hand, MSCs have immunomodulatory and anti-inflammatory capacity. Thus, the MSCs obtained according to the present invention can also be used in the treatment of diseases, where immunomodulatory or anti-inflammatory properties are needed as well as any disease, where a combination of those characteristics is useful.

Examples for indications where a treatment with MSCs is useful, are musculoskeletal/rheumatic disorders, the field of hematology/oncology, in particular the treatment of graft versus host disease (GvHD) and HSC graft enhancement, neurological disorders, gastrointestinal disorders and other indications. Regarding musculoskeletal/rheumatic disorders, MSCs predominantly are used for cartilage and bone repair or reconstructive surgery/tissue enhancement. Treatment of cardiovascular disorders includes treatment for decubitus and leg ulcers, where a cellular or engineered tissue therapy is necessary, or peripheral artery disease and heart failure. With regard to neurological diseases, multiple sclerosis and amyotrophic lateral sclerosis are examples. Gastrointestinal disorders include for example Crohn's disease. Other indications are in particular solid tumor and skin reconstruction.

Autologous cells are used for disorders such as musculoskeletal/rheumatic or cardiovascular disorders and for solid tumors, whereas allogeneic cells are predominant for the treatment in the field of hematology/oncology.

The MSC preparations obtained according to the present invention are useful for all indications. An important use of MSC is in the treatment of GvHD and cartilage or bone repair.

The MSCs obtained according to the present invention can also be used for reconstruction of skin, bone or cartilage, where a scaffold can be used to create a transplant.

### Definitions

In this description and the claims, reference will be made to a number of terms, which shall be defined to have the following meanings:
Mesenchymal stem cells or multipotent stromal cells (MSCs) are adult stem cells traditionally found in the bone marrow. However, mesenchymal stem cells can also be isolated from other tissues including cord blood, peripheral blood, fallopian tube, and fetal liver and lung. MSCs differentiate to form adipocytes, cartilage, bone, tendons, muscle, and skin.

Morphologically, MSCs have long thin cell bodies with a large nucleus. As with other stem cell types, MSCs have a high capacity for self renewal while maintaining multipotency. Thus, mesenchymal stem cells have enormous therapeutic potential for tissue repair. MSCs have been shown to be capable of differentiating ex vivo and in vivo into multiple cell types including adipocytes, chondrocytes, osteocytes, tenocytes, myocytes and specifically cardiomyocytes.

A cell can be classified as an MSC if it shows plastic adherent properties under normal culture conditions and has a fibroblast-like morphology. The cultured MSCs also express on their surface CD73, CD90 and CD105, while lacking the expression of CD11b, CD14, CD19, CD34, CD45, CD79a and HLA-DR surface markers. The bone marrow population includes a cell type, which is able to give rise in vitro to fibroblast colonies (CFU-F). CFU-F are adherent, nonphagocytic, nonproliferating, relatively radioresistant, la antigen-negative cells having a wide density and sedimentation rate distribution.

The majority of modern culture techniques take a colony-forming unit-fibroblasts (CFU-F) approach, where raw unpurified bone marrow or Ficoll^{®}-purified bone marrow mononuclear cells (MNCs) are plated directly into cell culture plates or flasks. MSC, but not red blood cells (RBCs) or hematopoetic progenitors, are adherent to tissue culture plastic within 24 to 48 hours.

The Total Nucleated Cell count or TNC is the test most often reported as a measure of the cell count after blood component separation. The main advantage of measuring TNC is that the count is highly reproducible within and among labs, so it can be used accurately throughout the blood banking community. Even better, the TNC count can be automated with the use of a device called a flow cytometer. However, the TNC count includes both living and dead cells

A "culture medium" is any liquid that can be used for growing MSCs, i.e. each culture medium suitable for culturing MSCs. An exemplary culture medium is Dulbecco's modified eagle medium (DMEM). DMEM can be supplemented as is common in this field, for example with L-Glutamine, antibiotics, bovine or human plasma and/or serum.

At present, measuring the percentage of CFU-F in the MSC sample is considered to be the best measure of whether the MSCs are viable. In the CFU-F test a small portion is watched under controlled conditions to see if MSCs divide and form colonies

### References

[1] Kim N, Cho S.G. Clinical applications of mesenchymal stem cells. The Korean Journal of Internal Medicine 2013;28(4):387-402. doi:10.3904/kjim.2013.28.4.387.
[2] Ikebe C. and Suzuki K., "Mesenchymal Stem Cells for Regenerative Therapy: Optimization of Cell Preparation Protocols," BioMed Research International, vol. 2014, Article ID 951512, 11 pages, 2014
[3] Kinya Ito K., Aoyama T., Fukiage K., Otsuka S., Furu M., Jin Y., Nasu A., Ueda M., Kasai Y., Ashihara E., Kimura S., Maekawa T., Kobayashi A., Yoshida S, Niwa H., Otsuka T., Nakamura, Toguchida. (2010) Novel Method to Isolate Mesenchymal Stem Cells from Bone Marrow in a Closed System Using a Device Made by Nonwoven Fabric, Tissue Engineering: Part C, 16: 81-91
[4] Goh, B. C., Thirumala, S., Kilroy, G., Devireddy, R. V. and Gimble, J. M. (2007), Cryopreservation characteristics of adipose-derived stem cells: maintenance of differentiation potential and viability. J Tissue Eng Regen Med, 1: 322-324 abstract (http://onlinelibrary.wiley.eom/doi/10.1002/term.35/abstract)

### List of reference signs

The following reference signs are used in the drawing and in the description.
(1) Injection port
(2) Filter
(3) Sample vessel
(4) Wash vessel
(5) Separation column
(6) Waste vessel for used wash solution
(7) Harvest vessel
(8) Expansion vessel
(9) Ultra-filtration column
(10) Waste vessel for excess liquid removed from the sample during ultra-filtration
(11) MSC vessel for collecting the isolated and concentrated MSCs

### Examples

Preferred embodiments of the invention are outlined in the following examples, which should not be interpreted as restricting the scope or spirit of the invention.

### Example 1: MSCs isolation capacity of different isolation principles.

15 ml bone marrow is withdrawn by means of a sterile disposable trocar from the right Posterior Superior Iliac Spine area, and 15 ml from the left PSIS area, into heparinized syringes. One third of the dose (10 ml) is then used for MSC isolation by means the RegenKit^{®}, Biomet BIOCUE^{™} and Kaneka separation systems. These exploits different principles for cell isolation, namely
- RegenKit^{®}: physical barrier (thixotropic gel).
- Biomet BIOCUE^{™}: dual buoy design with liquids.
- Kaneka: physico-chemical interaction (affinity) with a polymeric nonwoven fabric filter.

The separation process is carried out following the procedures reported for the systems. The results are in Table 1 and shows a higher isolation yield for the system working according to the principle of physic-chemical affinity.

**Table 1**

| | MSC yield |
|---|---|
| RegenKit^{®} | 62 % |
| Biomet BIOCUE^{™} | 71 % |
| Kaneka | 88 % |

### Example 2: Bone marrow component separation and expansion (for comparison)

15 ml bone marrow is withdrawn by means of a sterile disposable trocar from the right Posterior Superior Iliac Spine (PSIS) area, and 15 ml from the left PSIS area, into heparinized syringes. Half dose (15 ml) is then used for MSC isolation and expansion.

MSCs are isolated from the bone marrow by means of the Bone Marrow MSC Separation Device (Kaneka Corporation), which works on the principle of physico-chemical affinity with a polymeric nonwoven fabric filter, following the instructions provided by the manufacturer. 0.9% saline solution is used for cell attachment on the column and washing of the cells. Dulbecco's modified eagle medium with 10% fetal bovine serum (FBS) is used for detaching the cells from the column and expanding them for 7 days.

The procedure is performed with the following experimental parameters:
- Priming of column: about 10 mL 0.9% sodium chlorine solution.
- Washing of cells: about 100 mL 0.9% sodium chlorine solution.
- Harvesting of cells: about 50 mL Dulbecco's modified eagle medium with 10% FBS.

MSCs are evaluated as cell number (hematology blood analyzer/Coulter counter and manual counting with hemocytometer), viability (flow cytometer) and clonogenic potential (culturing cells in monolayer for CFU-F assay).

### Example 3: Bone marrow component separation, expansion and concentration (according to the invention)

Half-dose (15 ml) as in Example 2 is used for isolation, expansion and concentration of MSCs by means of the system object of this invention as in Figure 1.

The procedure is performed as described above with the following experimental parameters:
- Priming of column: about 10 ml 0.9% sodium chloride solution.
- Injection of half-dose bone marrow
- Attachment of cells
- Washing of cells: about 100 ml 0.9% sodium chloride solution.
- Harvesting of cells: about 50 ml Dulbecco's modified eagle medium with 10% FBS
- Concentration of cells to obtain about 7 mL MSC concentration in medium

MSCs are evaluated as in Example 2.

The results of the Examples 2 and 3 are reported in the Table 2.

**Table 2**

| | Example 1 | Example 2 |
|---|---|---|
| Cell number | 2.5×10⁶ cells | 2.5×10⁶ cells |
| Cell concentration (suspension) | 5×10⁴ cells/ml | 35×10⁴ cells/ml |
| Recovery of TNC | 51% | 48% |
| Removal of RBC (%) | 98% | 98% |
| Recovery of CFU-F | 85% | 83% |
| Viability of TNC | >80% | >80% |
| Cell suspension volume | 50 ml | 7 ml |

The results show that there is no difference in viability and functionality between MSCs treated as in Example 2 and Example 3. The higher MSC concentration in Example 2 is due to the much lower final volume obtained by the additional concentration step.

### Example 4: Bone marrow component separation (for comparison)

15 ml bone marrow is withdrawn by means of a sterile disposable trocar from the right Posterior Superior Iliac Spine (PSIS) area, and 15 ml from the left PSIS area, into heparinized syringes. Half dose (15 ml) is then used for MSC isolation and expansion.

MSCs are isolated from the bone marrow by means of the Bone Marrow MSC Separation Device (Kaneka Corporation) following the instructions provided by the manufacturer. 0.9% saline solution is used for cell attachment on the column, washing of the cells and detaching the cells from the column.

The procedure is performed with the following experimental parameters:
- Priming of column: about 10 ml 0.9% sodium chloride solution.
- Washing of cells: about 100 ml 0.9% sodium chloride solution.
- Detaching cells: about 50 ml 0.9% sodium chloride solution.

MSCs are evaluated as cell number (hematology blood analyzer/Coulter counter), viability (flow cytometer) and clonogenic potential (culturing cells in monolayer for CFU-F assay).

### Example 5: Bone marrow component separation and concentration (according to the invention)

Half-dose as in Example 3 is used for isolation and expansion of MSCs by means of the system according to this invention as shown in Figure 1.

The procedure is performed in compliance with what reported above in the "Description of invention" with the following experimental parameters:
- Priming of column: about 10 ml 0.9% sodium chloride solution.
- Injection of half-dose bone marrow
- Attachment of cells
- Washing of cells: about 100 ml 0.9% sodium chloride solution.
- Harvesting of cells: about 50 ml Dulbecco's modified eagle medium with 10% FBS
- Concentration of cells to obtain about 7ml MSC concentrate in medium.

MSCs are evaluated as in Example 2.

The results of Examples 4 and 5 are reported in the Table 3.

**Table 3**

| | Example 3 | Example 4 |
|---|---|---|
| Recovery of TNC | 51% | 50% |
| Removal of RBC (%) | 98% | 98% |
| Recovery of CFU-F | 85% | 83% |
| Viability of TNC | >80% | >80 |
| Cell suspension volume | 50 ml | 7 ml |

The results show that there is no difference in viability and functionality between MSCs treated as in Example 4 and Example 5.

### Example 6: Comparison of different types of filtration

The cell isolation has been performed in duplicate as in Example 4. After harvesting of cells each cell suspension has been concentrated with hollow fiber filters by the means of:
A. Dead-end filtration.
B. Tangential-flow filtration.

MSCs are evaluated as in Example 2.

The results are in Table 4.

**Table 4**

| | A | B |
|---|---|---|
| Recovery of TNC | 88% | 70% |
| Recovery of CFU-F | 87% | 67% |
| Viability of TNC | >80% | <75% |
| Cell suspension volume | 7 ml | 7 ml |

The results show that dead-end filtration provides a superior mean of cell concentration with hollow fiber filters.

## Claims

1. System for isolating multipotent stromal cells (MSCs), the system comprising
a) a separation unit comprising a separation column (5) comprising separation means in the form of a separation filter, separation beads or a separation gel for separating MSCs by physical and/or chemical affinity or physicochemical affinity, comprised in a sample, with a port for introduction of a sample, and an MSC outlet;
b) communication means for transferring an MSC rich fraction obtained from the separation unit to a concentration unit,
c) a concentration unit for concentrating the harvested MSCs; wherein the concentration unit is a hollow fiber dead-end ultrafiltration unit; and
d) an MSC receiving vessel;
wherein the system is a completely closed system.

2. System of claim 1, which additionally comprises an expansion unit and communication means connecting the expansion unit with separation unit and concentration unit within a completely closed system.

3. System of any one of the preceding claims, wherein the column comprising separation means by physical and/or chemical affinity or physicochemical affinity is a column at least partially covered by a filter fabric.

4. System of any one of the preceding claims, wherein the sample comprising MSCs is a biological fluid obtained from bone marrow, amniotic fluid, or umbilical cord blood.

5. Method of providing concentrated MSCs ready for administration to a patient using the system of any one of claims 1 to 4, wherein the method comprises
a) optionally priming a separation unit;
b) introducing a sample comprising MSCs into the separation unit;
c) washing the separation column and harvesting the MSCs;
d) optionally expanding the MSCs harvested in step c);
e) concentrating the fraction enriched with MSCs harvested in step c) in the concentration unit;
f) collecting the concentrated sample comprising MSCs.

6. Method of claim 5, wherein the step of expanding MSCs harvested from the separation column by physical and/or chemical affinity or physicochemical affinity in step c) prior to transferring the harvested MSCs into the concentration unit comprises growing MSCs in the expansion vessel, wherein the expansion vessel comprises a culture medium and culturing the harvested MSCS until a desired number of MSCs is obtained.

## Patentansprüche

1. System zur Isolierung von multipotenten Stromazellen (MSCs), umfassend
a) eine Trenneinheit, umfassend eine Trennsäule (5), die Trennmittel in Form eines Trennfilters, von Trennperlen oder eines Trenngels zur Trennung von MSCs durch physikalische und/oder chemische Affinität oder physikochemische Affinität umfasst, die in einer Probe enthalten sind, mit einer Öffnung zum Einführen einer Probe, und einem MSC-Auslass;
b) Kommunikationsmittel zum Umsetzen einer MSC-reichen Fraktion, die von der Trenneinheit erhalten wurde, zu einer Konzentrationseinheit,
c) eine Konzentrationseinheit zum Konzentrieren der entnommenen MSCs, wobei die Konzentrationseinheit eine Hohlfaser-Tot-End-Ultrafiltrationseinheit ist, und
d) ein MSC-Aufnahmegefäß;
wobei das System ein vollständig geschlossenes System ist.

2. System nach Anspruch 1, das zusätzlich eine Expansionseinheit und Kommunikationsmittel umfasst, das die Expansionseinheit mit Trenneinheit und Konzentrationseinheit innerhalb eines vollständig geschlossenen Systems verbinden.

3. System nach einem beliebigen der vorhergehenden Ansprüche, wobei die Säule, die Trennmittel durch physikalische und/oder chemische Affinität oder physikalisch-chemische Affinität umfasst, eine Säule ist, die mindestens teilweise mit einem Filtergewebe abgedeckt ist.

4. System nach einem der vorangehenden Ansprüche, wobei die MSCs umfassende Probe eine biologische Flüssigkeit ist, die aus Knochenmark, Fruchtwasser oder Nabelschnurblut erhalten ist.

5. Verfahren zum Bereitstellen von konzentrierten MSCs, die für die Verabreichung an einen Patienten bereit sind, unter Verwendung des Systems nach einem der Ansprüche 1 bis 4, wobei das Verfahren aufweist:
a) vorzugsweise das Vorbereiten einer Trenneinheit;
b) Einführen einer MSCs umfassenden Probe in die Trenneinheit;
c) Waschen der Trennsäule und Ernten der MSCs;
d) vorzugsweise Expandieren der in Schritt c) entnommenen MSCs;
e) Konzentrieren der mit in Schritt c) entnommenen MSCs angereicherten Fraktion in der Konzentrationseinheit;
f) Sammeln der konzentrierten Probe, die MSCs umfasst.

6. Verfahren nach Anspruch 5, wobei der Schritt zum Expandieren von von der Trennsäule entnommenen MSCs durch physikalische und/oder chemische Affinität oder physikalisch-chemische Affinität in Schritt c) vor dem Umsetzen der entnommenen MSCs in die Konzentrationseinheit ein Züchten von MSCs in dem Expandiergefäß, wobei das Expandiergefäß ein Kulturmedium umfasst, und Kultivieren der entnommenen MSCs, bis eine gewünschte Anzahl von MSCs erhalten wird, aufweist.

## Revendications

1. Système pour isoler des cellules stromales pluripotentes (MSC), le système comprenant :
a) une unité de séparation qui comprend une colonne de séparation (5) qui comprend des moyens de séparation sous la forme d'un filtre de séparation, de billes de séparation ou d'un gel de séparation pour séparer les MSC par affinité physique et/ou chimique ou par affinité physicochimique, lesquelles cellules sont incluses dans un échantillon, comprenant un orifice pour introduire un échantillon et une sortie de MSC ;
b) des moyens de communication pour transférer une fraction riche en MSC obtenue depuis l'unité de séparation jusqu'à une unité de concentration ;
c) une unité de concentration pour concentrer les MSC récoltées, dans lequel l'unité de concentration est une unité d'ultrafiltration sans issue à fibre(s) creuse(s) ; et
d) une cuve de réception de MSC ;
dans lequel le système est un système complètement fermé.

2. Système selon la revendication 1, lequel comprend de façon additionnelle une unité de développement et des moyens de communication qui connectent l'unité de développement à l'unité de séparation et à l'unité de concentration à l'intérieur d'un système complètement fermé.

3. Système selon l'une quelconque des revendications précédentes, dans lequel la colonne qui comprend des moyens de séparation par affinité physique et/ou chimique ou par affinité physicochimique est une colonne au moins partiellement recouverte par un tissu filtrant.

4. Système selon l'une quelconque des revendications précédentes, dans lequel l'échantillon qui comprend les MSC est un fluide biologique qui est obtenu à partir de la moelle osseuse, du fluide amniotique ou du sang du cordon ombilical.

5. Procédé de fourniture de MSC concentrées prêtes pour une administration à un patient en utilisant le système selon l'une quelconque des revendications 1 à 4, dans lequel le procédé comprend :
a) en option, l'amorçage d'une unité de séparation ;
b) l'introduction d'un échantillon qui inclut des MSC à l'intérieur de l'unité de séparation ;
c) le lavage de la colonne de séparation et la récolte des MSC ;
d) en option, le développement des MSC récoltées au niveau de l'étape c) ;
e) la concentration de la fraction enrichie avec les MSC qui sont récoltées au niveau de l'étape c) dans l'unité de concentration ; et
f) la collecte de l'échantillon concentré qui inclut les MSC.

6. Procédé selon la revendication 5, dans lequel l'étape de développement des MSC qui sont récoltées à partir de la colonne de séparation par affinité physique et/ou chimique ou par affinité physicochimique au niveau de l'étape c) avant le transfert des MSC récoltées à l'intérieur de l'unité de concentration comprend la croissance des MSC dans la cuve de développement, dans lequel la cuve de développement comprend un milieu de culture, et la mise sous-culture des MSC récoltées jusqu'à ce qu'un nombre souhaité de MSC soit obtenu.
